Europäisches Patentamt

European Patent Office

Office européen des brevets

(11) Publication number: **0 267 703 B1**

(12) **EUROPEAN PATENT SPECIFICATION**

(45) Date of publication of patent specification: **19.06.91**

(51) Int. Cl.⁵: **C12N 15/62**, C12N 1/20, C12P 21/02, C07K 13/00, A61K 37/02

(21) Application number: **87309318.1**

(22) Date of filing: **21.10.87**

(54) Human apolipoprotein AI and variant form of the same expressed in Escherichia coli.

(30) Priority: **23.10.86 GB 8625435**

(43) Date of publication of application:
**18.05.88 Bulletin 88/20**

(45) Publication of the grant of the patent:
**19.06.91 Bulletin 91/25**

(84) Designated Contracting States:
**AT BE CH DE ES FR GB GR IT LI LU NL SE**

(56) References cited:
**WO-A-86/04920**

**FEBS, vol. 194, no. 2, January 1986, pages 343-346; Federation of European Biochemical Societies; R. LORENZETTI et al.: "Expression of the human apolipoprotein AI gene fused to the E. Coli gene for beta-galactosidase"**

**PATENT ABSTRACTS OF JAPAN, vol. 10, no. 276 (C-373)[2332], 19th September 1986; & JP-A-61 96 998 (MITSUBISHI CHEM IND LTD) 15-05-1986**

(73) Proprietor: **FARMITALIA CARLO ERBA S.r.L.**
**Via Carlo Imbonati 24**
**I-20159 Milano(IT)**

(72) Inventor: **Lorenzetti, Rolando**
**Via Felice Cavallotti, 137**
**I-20152 Monza(IT)**
Inventor: **Monaco, Lucia**
**Via Cola di Rienzo, 51**
**I-20100 Milan(IT)**
Inventor: **Soria, Marco**
**Via Guerrini 13**
**Milan(IT)**
Inventor: **Palomba, Raffaele**
**Via Fornari 46**
**Milan(IT)**
Inventor: **Isacchi, Antonella**
**Viale Lazio 27**
**Milan(IT)**
Inventor: **Sarmientos, Paolo**
**Via M. Bianchi 24**
**Milan(IT)**

THE EMBO JOURNAL, vol. 6, no. 11, November 1987, pages 3253-3260, IRL Press LTD, Oxford, GB; L. MONACO et al.: "A recombinant apoA-1-protein A hybrid reproduces the binding parameters of HDL to its receptor"

NATO ADVANDEED STUDY INSTITUTE SERIES, SERIE A, LIFE SCIENCES, vol. 112, 1986, pages 153-160; L. MONACO et al.: "Versatile E.coli expression-modification vectors as tools for structure-function studies of apolipoprotein A1"

(74) Representative: **Woods, Geoffrey Corlett et al J.A. KEMP & CO. 14 South Square Gray's Inn London WC1R 5EU(GB)**

## Description

The present invention relates to the production of human apolipoprotein AI (apoAI) and its variants by means of recombinant DNA technology.

Atherosclerosis, and its attendant complications (i.e. coronary heart disease, CHD), is one and perhaps the most critical health problem in the world. A great number of risk factors have been associated with the development of the disease, one of the most important of these being elevated plasma cholesterol (CHL) levels. Thus, a great deal of attention has been devoted to the study of CHL metabolism in man.

The lipoprotein transport system holds the key to understanding the mechanisms by which genes, diet and hormones interact to regulate the plasma CHL and triglyceride (TG) levels in man. The function of the lipoproteins in plasma is mainly to transport lipids from one organ to another. It has recently become evident that they not only solubilize hydrophobic lipids, but may also dictate the site of the body to which each lipid class is to be delivered. There are four main classes of lipoproteins: chylomicrons (CM), very low density (VLDL), low density (LDL) and high density (HDL) lipoproteins.

For transport in plasma, TG and cholesteryl esters (CHLE) are packaged into lipoprotein particles in which they form a hydrophobic core surrounded by a surface monolayer of polar phospholipids (PL). The surface coat also contains unesterified CHL in relatively small amounts together with proteins called apolipoproteins (apo). At least nine apos have been identified: AI, AII, AIV, B (48-100), CI, CII, CIII, D and E.

Of particular interest is the investigation on the relations between plasma lipoprotein levels and the risk of development of CHD. Both HDL and LDL are carriers of CHL and CHLE; however there are indications that, while LDL-CHL levels are a positive risk factor (Kannel et al., Ann. Intern. Med., 90:85-91, 1979), HDL's are an important negative risk factor (Yaari et al., Lancet, i:1011-1015, 1981). Although the precise function and mode of action of these lipoproteins has not yet been completely understood, it appears that HDL serves particularly to remove CHL from peripheral tissues and transport it back to the liver, with a mechanism named reverse CHL transport (RCT).

The chief apolipoprotein of HDL is apoAI and several studies have shown an inverse association between plasma apoAI levels and CHD similar to that which has been documented for HDL-CHL levels and CHD (Ishikawa et al., Eur. J. Clin. Invest., 8:179-182, 1978). In addition, both HDL-CHL and apoAI levels are inversely correlated to the severity to angiographically demonstrable coronary arteriosclerotic lesions (Pearson et al., Amer. J. Epidem., 109:285-295, 1979; Maciejko et al., New Eng. J. Med., 309:385-389, 1983). Thus it can be argued that high concentrations of HDL in plasma influence CHD by retarding atherogenesis and/or promoting regression of existing lesions.

Complexes of HDL apolipoproteins, mainly apoAI, and lecithin promote the efflux of free CHL from cells in vitro, including cultured arterial smooth muscle cells (Stein et al., Biochem. Biophys. Acta, 380:106-118, 1975). Intravenous infusion of phospholipds into animals with experimentally-induced atheroma favourably influences this efflux (Adams et al., J. Path. Bact., 94:77-87, 1967), and apoAI/lecithin complexes are cleared from plasma at a rate similar to that of native HDL particles (Malmendier et al., Clin. Chem. Acta, 131:201-210, 1983).

The intravenous infusion of apoAI in CHL fed rabbits reduces the areas of lesion involvement by 50%. This indicates that apoAI has a protective effec t on atherosclerotic lesion formation, possibily by decreasing CHL uptake by the aortic wall (Maclejko and Mao, Artheriosclerosis, 2:407a, 1982; Mao et al., Fed. Proc. (USA), 42, no 7 pABSTRACT 357, 1983; Badimon et al., Cardiovascular Disease '86, 1986 ABSTRACT 81).

ApoAI is the major protein of HDL, about 70%, and is relatively abundant in plasma with a concentration of 1.0-1.2 mg/ml (Schonfield et al., J. Clin. Invest., 69:1072, 1982). Plasma apoAI is a single polypeptide chain of 243 amino acids whose primary sequence is known (Brewer et al., Biochem. Biophys. Res. Commun., 80:623-630, 1978). In its intracellular form apoAI is present as a 267 amino acid long precursor; the first 18 N-terminal residues of this longer protein are cleaved intracellularly by the signal peptidase of the rough endoplasmic reticulum. The newly excreted apoAI still contains a 6 amino acid long N-terminal extension; the conversion into its mature form is performed by a plasma and/or lymph specific protease (Zannis and Breslow, in Advances in Human Genetics, Harris and Hirschhorn eds., Plenum, 125-215, 1985).

The C-terminal part of the apoAI molecule has been shown. to consist of repeat units of 11 or 22 amino acids (McLachlan, Nature, 267:465-466, 1979); the tandemly arrayed segments are not exact duplications, however, but amino acid subsitutions have generally conserved the chemical type of residues in corresponding positions of the repeats. These repeat segments are presumed to exist in amphipathic helical conformation (Segrest et al., FEBS Lett., 38:247-253, 1974), believed to be the major structural requirement for the main biological activities of apoAI, i.e. lipid binding and lecithin CHL acyltransferase (LCAT) activation. Two other functions of apoAI, acting as ligand for the recognition of HDL particles by a receptor, and removal of CHL from peripheral tissues, have not yet been related to specific sequences or domains in the apoAl molecule.

The first described molecular variant of human apoAl is the Milano (apoAl-MI) mutant (Franceschini et al., J. Clin. Invest., 66:892-900, 1980). This is characterized by the Arg173-Cys substitution (Weisgraber et al., ( J. Biol. Chem., 258:2508-2513, 1983), and was identified in 33 subjects belonging to the same kindred. All of them were heterozygous for the mutant apoprotein, which is transmitted as an autosomal dominant trait. They showed markedly reduced HDL-CHL concentrations and hypertriglyceridemia of a variable degree, negatively correlated with the HDL levels. No association between the mutant and specific pathological conditions could be established; indeed the affected subjects were apparently protected from the development of early atherosclerosis (Gualandri et al., Am. J. Hum. Gen., 1986, in press).

The amino acid substitution in the apoAl-MI modifies the structure of the mutant apoprotein, leading to a reduction of ordered alpha-helical structure and to an increased exposure of hydrophobic residues (Franceschini et al., J. Biol. Chem., 260:16321-16325, 1985). The remodelling of the mutant apoprotein significantly alters the lipid binding properties of the molecule which associates more readily than normal apoAl with lipids. Apoprotein/lipid complexes are similar to those formed by normal apoAl but are more easily destroyed by denaturing agents. The presence of a cysteine residue in the variant form allows the formation of complexes with apoAll, as well as apoAl dimers; these protein complexes are probably responsible for the formation of he anomalous HDL particles observed in the "affected" subjects. All these features of apoAl-MI may contribute to its accelerated catabolism, as well as to an efficient uptake capacity for tissue lipids.

Several other genetic variants of apoAl have been described so far (Breslow, Ann. Rev . Biochem., 54:699-727, 1985), but none of these is associated with any pathological conditions or, in general, to significant alterations of lipoprotein metabolism in the carriers (Table 1 below).

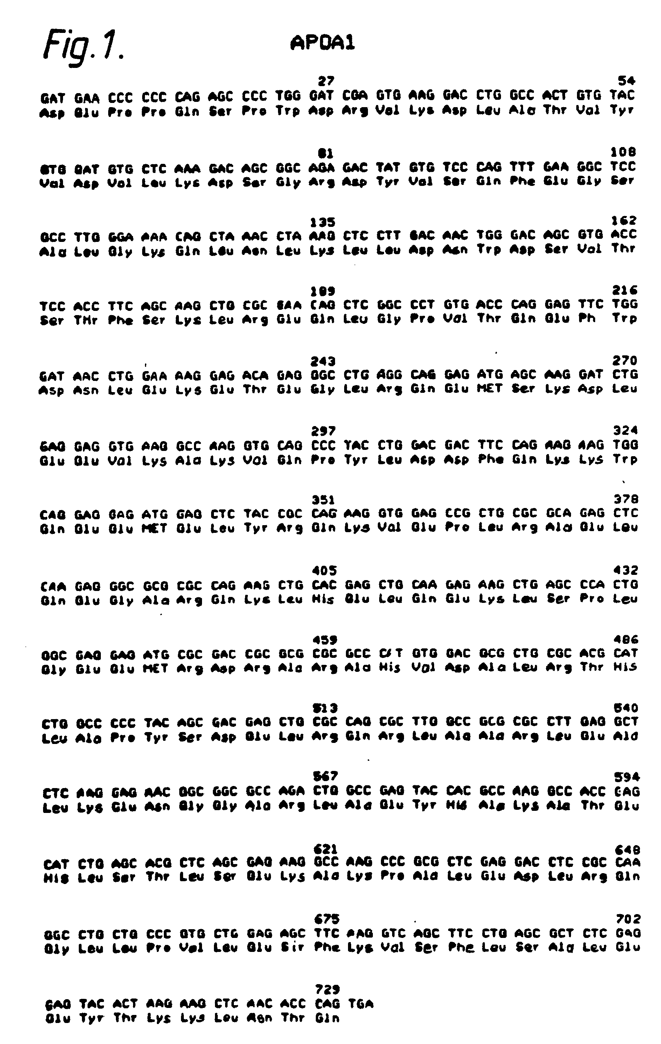

ApoAl cDNA clones have been obtained by several laboratories (Breslow, Ann. Rev. Biochem., 54:699-727, 1985; Sharpe et al., Nucl. Acids Res., 12:3917-3932, 1984). The mRNA is about 890 base pairs (bp) long and presents a 5' untranslated sequence of 35bp, a coding sequence of 801bp (267 amino acids), a translation termination codon (TGA) and a 3' untranslated sequence of 54bp followed by a polyA tail. The complete cDNA nucleotide sequence is shown in Figure 1 of the accompanying drawings. Monaco et al (Nato Advanced Study Institute Series, Series A, Life Sciences, vol. 112, 1986, 153-160) disclose a failed attempt to express mature apoAl based on an approach using a vector pFCE4+. Lorenzetti et al (FEBS Letters 194 (1986) 343-346) discloses the expression in E. coli of human apoAl fused to the beta-galactosidase protein.

We have now used recombinant DNA technology successfully to produce proteins comprising apoAl or a genetic variant thereof fused to a short N-terminal sequence derived from beta-galactosidase or to the pro-sequence of apoAl. The variant maybe apoAl in which the 6-Ser has been replaced by Thr (apoAl-T6), apoAl-MI or a variant which combines the mutations of both apoAl-T6 and apoAl-MI (apoAl-T6/MI). The proteins can be detected by enzyme-linked immunoabsorbent assay (ELISA) with anti-apoAl anti-serum.

Accordingly, the present invention provides an expression vector capable of expressing, in a transferred host, a protein which is capable of being detected by ELISA with anti-human apoAl antiserum and which has the formula (1)

Met-X-Y   (1)

wherein X is -Thr-Met-Ile-Thr-Pro-Ser-Phe-Asp-Gly-Ser-Met-,-Thr-Met-Ile-Thr-Asn-Ser-Arg-Gly-Ser-Met-or the pro sequence of human apoAl; and Y represents the sequence of human apoAl or human apoAl in which the Arg at position 173 is substituted by Cys (apoAl-MI), in which the Ser at position 6 is substituted by Thr (apoAl-T6) or which incorporates both substitutions (apoAl-T6/MI). The invention also provides a process for producing a protein of formula (1), which process comprises:

(i) culturing a host transformed with such an expression vector capable of expressing the said protein in the said host; and

(ii) recovering the said protein thus obtained.

The Met residue is attributable to the translational start codon. The invention also provides hosts transformed with the expression vector and in which thereby a said protein is capable of being expressed. The transformed host is cultured and the protein of formula (1) thus obtained is recovered. The proteins of formula (1) also form part of the invention.

In preferred embodiments, the invention relates to A) the construction of recombinant plasmids bearing the genes for:

1-apoAl with the following N-terminal extension Thr-Met-Ile-Thr-Pro-Ser-Phe-Asp-Gly-ser-Met- (apoAl-

4

RP5),

2-apoAI with the following N-terminal extension Thr-Met-Ile-Thr-Asn-Ser-Arg-Gly-ser-Met- (apoAI-IPI),

3-the protein which combines apoAI-T6 and apoAI-RP5 (apoAI-RP5/T6),

4-the protein which combines apoAI-T6 and apoAI-IPI (apoAI-IPI/T6),

5-the protein which combines apoAI-MI and apoAI-RP5 (apoAI-RP5/MI),

6-the protein which combines apoAI-MI and apoAI-IPI (apoAI-IPI/MI),

7-the protein which combines apoAI-T6/MI and apoAI-RP5 (apoAI-RP5/T6/MI),

8-the protein which combines apoAI-T6/MI and apoAI-IPI (apoAI-IPI/T6/MI),

9-apoAI with the following N-terminal extension Arg-His-Phe-Trp-Gln-Gln (proapoAI), and

10-the protein which combines proapoAI and apoAI-MI (proapoAI-MI); and

B) the expression of these genes in Escherichia coli (E. coli) strains. By expression of these genes, the following proteins are obtained:

- a fusion protein wherein the amino acid sequence Met-Thr-Met-Ile-Thr-Pro-Ser-phe-Asp-Gly-Ser-Met- is fused to an apolipoprotein selected from apoAI, apoAI-T6, apoAI-MI and apoAI-T6/MI;
- a fusion protein wherein the amino acid sequence Met-Thr-Met-Ile-Thr-Asn-Ser-Arg-Gly-Ser-Met is fused to an apolipoprotein selected from apoAI, apoAI-T6, apoAI-MI and apoAI-T6/MI; and
- a proapolipoprotein Met-proapoAI or Met-proapoAI-MI.

Preferably the protein of formula (1) in which X is: -Thr-Met-Ile-Thr-Pro-Ser-Phe-Asp-Gly-Ser-Met-is obtained by culturing a host transformed with plasmid pUC8 having the gene encoding apoAI, apoAI-T6, apoAI-MI or apoAI-T6/MI provided at the Bam HI site immediately after the codon for the ninth amino acid of the alpha-peptide of the beta-galactosidase gene of the said plasmid.

Preferably the protein of formula (1) in which X is:

-Thr-Met-Ile-Thr-Asn-Ser-Arg-Gly-Ser-Met-

is obtained by culturing a host transformed with plasmid pUC9 having the gene encoding apoAI, apoAI-T6, apoAI-MI or apoAI-T6/MI provided immediately after the codons for the sequence:

-Thr-Met-Ile-Thr-Asn-Ser-Arg-Gly-Ser-Met-

which are in turn provided immediately after the translational start codon provided by the plasmid.

Preferably the protein of formula (1) in which X is the pro sequence of human apoAI is expressed from a plasmid under the control of the trp promoter.

In the accompanying drawings:

Figure 1 shows the complete nucleotide sequence corresponding to the mature form of human apoAI. The sequence is shown in the mRNA sense in the 5' to 3' direction; the lower line shows the amino acid sequence.

Figure 2 shows the reconstruction of the 5' end of the gene coding for mature human apoAI. A DNA fragment from plasmid pAI/A, starting at the first Sau 3AI restriction site, was ligated to a synthetic oligonucleotide adapter assembled by annealing and ligating the single-stranded precursors 1-4. The resulting 790 bp fragment, flanked by two Bam HI restriction sites, codes for the mature apoAI molecule.

Figure 3 relates to the nucleotide sequence of pLS66 and pML11-20. The sequences show the junction between the ATG start codon of pFCE4[+] and the beginning of the apoAI gene before (pLS66, A) and after the deletion (pML11-20, B).

Figure 4 relates to the nucleotide sequence of pIL8-6 and pIL8-I. The sequences show the presence of the apoAI-MI mutation (C to T) in pIL8-I (A) and of the apoAI-T6 mutation (G to C) in pIL8-6 (B).

Figure 5 shows immunoblot analysis of pML11-20. Aliquots of the eluted material out of the affinity column and of a standard human HDL fraction were boiled and electrophoresed in duplicate on a 12.5% SDS-PAGE. Blotting on nitrocellulose filter was performed as described in the text. Lanes: 1- standard HDL; 2- eluate from the affinity column; 3- $M_r$ standards.

Figure 6 shows immunoblot analysis of pRP5 and pUC9. Aliquots of bacterial cultures, bearing the wild type pUC9 or the recombinant pRP5 plasmids, induced with IPTG, were pelletted and resuspended in the appropriate buffer for gel electrophoresis in parallel with a standard human HDL fraction; after boiling samples were eletrophoresed on 12.5% SDS-PAGE. Blotting on nitrocellulose filter was performed as described in the text. Lanes: 1- $M_r$ standards; 2- standard HDL; 3- pUC9; 4-pRP5.

Figure 7 shows the reconstruction of the 5' proapoAI sequence.

Figure 8 shows the construction of vector pFC33 which is capable of expressing proapoAI.

Figure 9 shows a gel electrophoresis and immunoblot analysis of the proapoAI expressed by pFC33. Lanes 1: HDL standard, Lanes 2: Non-recombinant strain, Lanes 3: proapoAI and Lane 4: Molecular Weight Standard.

An expression vector according to the invention is a vector capable of expressing DNA sequences and, in particular, the sequences of structural genes which the vector contains. The DNA sequences to be expressed are correctly positioned in relation to the upstream sequences which control their expression. Any suitable promoter system may be employed taking into account the protein to be expressed, the nature of the host, etc. Thus, a promoter selected from the $P_L$, lac, tac and trp promoters may be employed. In the present instance, the 5'-regulatory sequences are heterologous with respect to the apoAI or genetic variant thereof to be expressed. The regulatory sequences may be those of the lac or trp operon. In other words, an expression vector of the invention is capable of expressing a heterologous protein wherein the heterologous protein is apoAI or a genetic variant thereof. The genetic variants are apoAI-T6, apoAI-MI and apoAI-T6/MI.

The expression vectors of the present invention, containing a DNA sequence encoding a protein of formula (1), are typically plasmids. They may be single-stranded. Each vector is replicable, containing origin of replication. Preferred expression vectors are derived from pUC8 or pUC9 in the case of expression of a fusion protein where the carrier sequence
is -Thr-Met-Ile-Thr-Pro-Ser-Phe-Asp-Gly-Ser-Met-
or -Thr-Met-Ile-Thr-Asn-Ser-Arg-Gly-Ser-Met-and pDS20 where Met- proapoAI or a genetic variant thereof is being expressed.

In order to express the protein comprising apoAI or a genetic variant thereof, a suitable host is transformed with an expression vector according to the invention. A procaryotic or eurcaryotic host may be employed such as bacteria, yeast or a mammalian cell line. Typically, the host is a strain of E. Coli. A host should be chosen in which the desired protein is not degraded by cellular proteases. In general terms, therefore, a protein comprising apoAI or a genetic variant thereof may be obtained according to the following procedure:

1. Cloned apoAI cDNA is selected.

2. The cDNA is tailored so that the apoAI gene is provided with restriction sites at both ends so it becomes readily movable. This may entail removing the sequences coding for the signal peptide and propeptide.

3. The apoAI gene is inserted in an expression vector in the correct reading frame. Mutations T6 and/or MI may be introduced. The apoAI gene or variant gene may be the sole struotural gene in an operon. Alternatively, it may be provided attached at its 5'-end to a DNA sequence encoding a carrier peptide where it is desired to express the apoAI or genetic variant thereof as a fusion protein.

4. A host in which the apoAI or genetic variant thereof is capable of being expressed is transformed with the expression vector.

5. The transformed host is cultured and the desired protein thus obtained is recovered.

Where a gene encoding apoAI or a genetic variant thereof is available without one or more introns, this gene may be inserted in frame in a vector under the transcriptional control of a promoter provided in the vector. The gene may be inserted immediately after a DNA sequence encoding a carrier peptide sequence. A DNA sequence encoding a protein of formula (I) can therefore be provided between translational start and stop codons in a vector providing the suitable promoter. Similarly, a gene encoding proapoAI or a genetic variant thereof can be inserted in a vector. Culture of a host transformed with the resultant expression vector leads to production of the desired protein.

The carrier peptide portion of a fusion protein may be derived from N-terminal amino acid residues of beta-galactosidase. Carrier peptides obtained in this way are the sequences:
Thr-Met-Ile-Thr-Pro-Ser-Phe-Asp-Gly-Ser-Met or
Thr-Met-Ile-Thr-Asn-Ser-Arg-Gly-Ser-Met

Recombinant proteins of formula (I) are typically provided essentially free of other proteins of human origin. In other words, a protein is provided in essentially pure form, unaccompanied by protein with which it is ordinarily associated.

The protein comprising apoAI or a genetic variant thereof may be employed in a method of treatment of the human or animal body by therapy. More particularly, it may be used to lower plasma cholesterol and/or triglyceride levels. Thus, a protein may be used to combat atherosclerosis and cardio-vascular diseases such as coronary heart disease. The protein may be administered preventatively or for the amelioration/cure of an existing condition.

A protein produced according to the invention may therefore be provided as a pharmaceutical composition also comprising a pharmaceutically active carrier or diluent. Such compositions may be formulated in known manner. The protein may be adminstered parenterally, for example intravenously.

The following Examples illustrate the invention.

Example 1

6

MATERIALS AND METHODS

Strains and Media

Strains: E. Coli K12 JM101 (Messing et al., Nature, 314:309-321, 1981), MC 1061 (Casadaban and · Cohen, J. Mol. Biol. 138:179-207, 1980), 71/18cl857 (Lorenzetti et al., Gene, 39:85-87, 1985), RL841 ( Kenkel, Proc.Natl. Acad.Sci.USA, 82:488-492, 1985), CAG629 ( rpoH165, lon-; C.A. Gross, Madison, Wisconsin).

Media: All strains were grown in LB medium with 50 ug/ml ampicillin when needed (LA medium). 1 mM IPTG was added to media to derepress the lac promoter when appropriate.

Enzymes, enzymatic reactions and DNA purification

Lysozyme was from Sigma (St. Louis, MO, USA). Restriction endonucleases were from Boehringer (Mannheim, DE), BRL Inc. (Gaithesburg, MD, USA) or New England Biolabs (Beverly, MA, USA); T4 ligase and all other enzymes were from Boehringer. All enzymatic reactions were performed according to the supplier's instructions unless otherwise specified. DNA fragments obtained by restriction endonuclease digestion were separated by electrophoresis in agarose horizontal gels or polyacrylamide vertical gels and extracted from the gel matrix by a modification of the electroelution method, using an ISCO Mod. 1750 concentrator (ISCO Co., Lincoln, NE, USA) and further purification on Elutip-d columns (Schleicher and Schuell, Keene, NH, USA).

Enzyme immunoassay for apoAl recognition in bacterial extracts (ELISA)

Pelleted cells were resuspended in lysis buffer (50 mM Tris-C1 pH 7.0, 30 mM NaCl, 0.1% NaN$_3$, 10 mg/l PhenylMethyl Sulfonyl Fluoride). The solution was made 1 mg/ml with lysozyme and was stirred at 4°C for 30 min. After 5 freeze-thawing cycles, the extracts were subjected to enzyme immunoassay as follows.

A standard 96 well microtiter plate was coated with appropriate dilutions of anti-human apoAl antiserum raised in sheep (Boehringer, Mannheim, DE) overnight at 4°C. After 3 washes with 10mM Tris-C1 pH 8.0 containing 0.05% Tween-20 and 0.01% Merthiolate, 50 μl of serial dilutions of apoAl standard (TAGO Corp., Burlingame, CA, USA) or of bacterial extract were added and incubated for 1 h at 37°C. After 3 washes with the same buffer, 50 μl were added of an appropriate dilution of rabbit anti-human apoAl antiserum (Immuno Ltd., Dunton Green, Nr. Sevenoaks, Kent, GB) further purified by ammonium sulfate precipitation. After 1 hr at 37°C and 3 more washes, the wells were stained with a protein A-horseradish peroxidase kit supplied by New England Nuclear, (Boston, MA, USA) according to the instructions of the manufacturer.

Coupling of sheep anti-human apoAl to Affigel 10

Preparation of the sheep and anti-apoAl antibody (Boehringer, Manneheim, DE): 20 ml of undiluted antibody solution were dialyzed against 0.1 M Phosphate buffer pH 7, overnight at 4°C stirring continuously; then all debris were spun down saving the supernatant.

Preparation of Affigel 10 (Bio-Rad, Richmond, CA): 40 ml were equilibrated at room temperature before repeated (4-5 times) cycles of washing with half volume of isopropyl alcohol and then with double distilled water; the gel was dried down by filtration and transferred to a 50 ml falcon tube adding the dialyzed antibody. The tube was then incubated at 4°C for 8 hours stirring continuously and 1 ml of 1 M glycine ethyl ester was added; incubation continued overnight. The gel was then extensively washed with the following solutions:

```
- 100 ml of PBS + 0.1% sodium azide
- "    "    "  1 M acetic acid
- "    "    "  0.1 M phosphate buffer pH 7 + 0.5 M
NaCl
- "    "    "  PBS + 0.1% sodium azide;   ·
```

packed into a column and equilibrated with the loading buffer (PBS + 10mg/l PMSF + 10 mg/l Pepstatin A).

Immunoblot analysis

Samples were run on polyacrylamide slab gels (at the appropriate concentration) using the Laemmli method. Then the gels were transferred on nitrocellulose filters using a transblot apparatus (Bio-Rad) at 0.2 A for 4 hours at 4°C in 25 mM Tris Base, 192 mM Glycine, 20% Methanol. After transfer the filters were washed with distilled water and then incubated 1 hour with PBS + 3% BSA with gentle shaking. Filters were washed again with distilled water and incubated with the first antibody (the same used in ELISA as second antibody) diluted 1:250 with PBS, and incubated 1 hour at room temperature. After 2 washes with PBS and PBS + 0.05% Tween 20 the filters were incubated with the second antibody (anti rabbit IgG raised in goat conjugated with horseradish peroxidase; Bio-Rad) diluted 1:7500 for 1 hour at room temperature. After two more washes, filters were stained with 4-chloro-1-napthol.

RESULTS

5′ end reconstruction of the apoAI gene

A full-length cDNA clone coding for apoAI (pAI/A) was obtained from a human liver cDNA library having the coding sequences for the signal peptide, the propeptide and the whole mature apoAI protein (Sharpe et al., Nucl. Acids Res., 12:3917-3932). We reconstructed the 5′ end of the gene such that all sequences upstream of the first codon of mature apoAI were removed. For this purpose, plasmid pAI/A was digested with EcoRI and BamHI and an 890 bp fragment containing the cDNA insert was isolated and recovered from a 1% agarose gel. The fragment was restricted with Sau3AI under partial digestion conditions, and the mixture was ligated with a phosphorylated synthetic adapter which had been designed to reconstruct the coding sequence for the first 9 amino acids of the mature protein, optimizing the sequence according to codon usage in E. coli. The adapter was prepared as follows: four oligonucleotides were synthesized by the phosphotriester method (Crea and Horn, Nucl. Acids Res., 8:2231-2348, 1980).

1. 5′ -GATCCATGGACGAGCC-3′
2. 5′ -ACCGCAGAGTCCATGG-3′
3. 5′ -CGGTGGCTCGTCCATG-3′
4. 5′ -GATCCCATGGACTCTG-3′

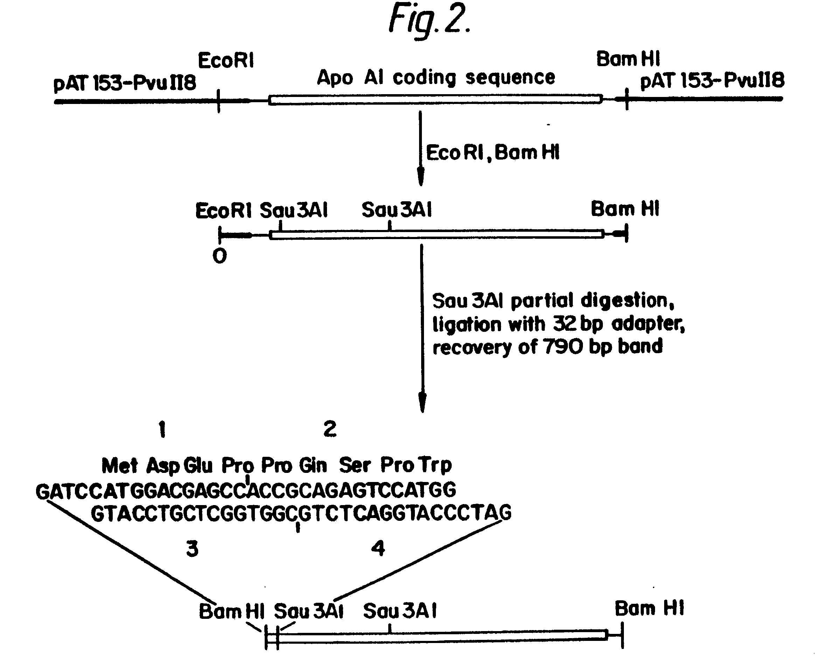

All four oligonucleotides were ligated together in equal molar amounts, the ligation mixture was treated with Sau3AI and the adapter was eluted and purified from a polyacrylamide gel. The resulting sequence of the adapter is illustrated in Fig. 2.

The ligation mixture containing the purified adapter and the partial Sau3AI-cut apoAI fragment was digested with BamHI. A 790 bp fragment was purified from a polyacrylamide gel and ligated to BamHI-cut pAT153-Pvull8 treated with Calf Intestine Phosphatase. After transformation of MC 1061, recombinants were screened by hybridization with phosphorylated oligonucleotide 3 as a probe. Positive colonies were isolated and further charaterised by restriction enzyme digestion and sequencing by partial chemical degradation according to Maxam and Gilbert ( Methods Enzymol., 65:499-560, 1980). The plasmid having the adapter in the correct orientation was designated pAI/12.

Thus reconstructed, the 5′ end of the apoAI gene was preceeded by an ATG start codon and a BamHI cohesive end. Using this strategy, the mature apoAI coding sequence could be moved to any expression vector carrying a BamHI site after the Shine-Dalgarno sequences.

Construction of recombinant vectors using pFCE4 +

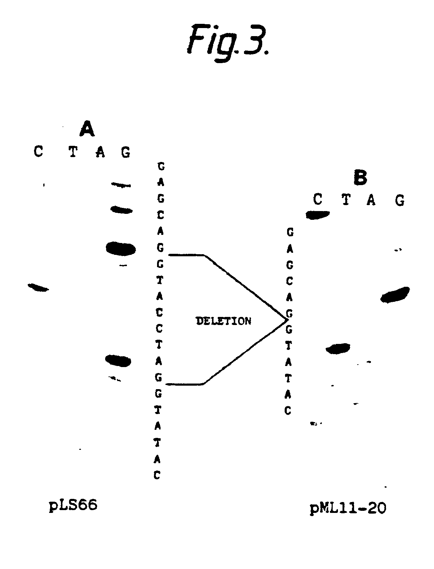

The BamHI fragment containing the modified apoAI gene was purified and subcloned into the BamHI site of pFCE4 + (Lorenzetti et al., 1985). The resulting construction with the correct orientation of the insert was verified by the dideoxy chain termination sequencing (fig.3A). This plasmid was named pLS66.

Using this strategy, the apoAI gene was inserted out of frame with respect to the ATG start codon already present in the vector. Thus, the next step was to restore the reading frame by positioning the apoAI gene immediately after this ATG codon.

In-frame positioning

Using the pFCE4 system the in-frame positioning of a cloned gene can be performed using "bridge" oligonucleotides, having sequences complementary to the stretches of bases flanking the region to be deleted on either side (Sollazzo et al., Gene, 37: 199-206, 1985).

To do so, we synthesized, by the phosphotriester method, a single-stranded oligonucleotide having the following sequence:

5' -CTTACATATGGACGAGCC-3'.

This oligonucleotide should anneal with its 5, end to the region immediately preceding and including the ATG of the vector (nucleotides 1 to 10), and with its 3' end to the first 8 nucleotides of the apoAI gene, thus looping out the 8 extra nucleotides present in pLS66 (Fig. 3). The ssDNA of pLS66 was prepared as described (Kunkel, Proc. Natl. Acad. Sci. USA, 82: 488-492, 1985) using E. coli RL841 as host. This bacterial strain is a derivative of E. coli RZ1032 containing plasmid pCl857 (M. Zabeau) which produces a temperature sensitive repressor for the $P_L$ promoter present in pFCE4.

Using this protocol, the ssDNA produced by this strain contained several uracil residues in place of thymine, thus acting as a normal functional template "in vitro" but not being biologically active upon transformation into a wild-type (ung +) E. coli 71/18cl857 strain, which is the normal host for these plasmids.

0.1 pmoles of pLS66 ssDNA were annealed with 2 pmoles of kinased mutagenic oligonucleotide (a 20 fold excess) at 56°C for 30 min in 15 mM Tris-C1, pH 8.5, and 15 mM MgC1$_2$. After cooling to room temperature the elongation-ligation mixture was added and the reaction was continued at 15°C overnight. The elongation reaction mixture was as follows (final concentrations): 10 mM Tris-C1, pH 8.5; 10mM MgC1$_2$; 0.5 mM ATP; 0.05 mM dNTPs; 5 mM DTT; 1 unit of DNA polymerase, Klenow fragment and 1.5 units of T4 DNA ligase.

After incubation the reaction mixture was phenol extracted, isopropanol and ethanol precipitated; 71/18cl1857 competent cells were transformed and plated on selective medium, LA. In order to identify clones that contained the desired deletion, 150 colonies were picked, grown on ordered plates and transferred onto nitrocellulose filters. The hybridization was performed at room temperature in 6× SSC and 10× Denhardt's using the mutagenic oligonucleotide as a probe. The filters were then washed in 6× SSC and 0.1% SDS at two different temperatures: the first time at room temperature, to check for hybridization efficiency, and then at 50°C, i.e. 4°C below the theoretical melting temperature. This was calculated according to the following formula, $Tm = 4 \times GC + 2 \times AT$, where GC and AT are the numbers of pairings formed by the oligonucleotide with the mutagenized template (Norrander et al., Gene, 26: 101-106, 1983). After each washing the filters were exposed with X-ray sensitive films. Candidate mutant colonies, yielding positive signals also at a stringent temperature, were confirmed by dideoxy chain-termination sequencing (Fig. 3B). The efficiency of this protocol is rather high, about 50%. pFCE4 + with the apoAI gene correctly positioned in frame was named pML11-20.

Construction of apoAI variants T6 and MI

Both pLS66 and pML11-20 were used as templates for the construction of the two variants apoAI-T6 and apoAI-MI. We decided to use both templates to obtain at the same time, the variant genes correctly positioned in frame and in a transposable BamHI fragment.

For this purpose two single oligonucleotides were synthesized by the usual protocol:

1. 5' -CACCGCAGACTCCATGG-3' for the T6 mutation
2. 5' -GCGCCAGTGCTTGGC-3' for the MI mutation

Indicating with number 1 the first nucleotide of the first codon of Mature apoAI, oligonucleotide 1 should anneal from residues 8 to 24, with a mismatch in position 17 (G to C). Oligonucleotide 2 shoud anneal from residues 510 to 524, with a mismatch in position 517 (C to T).

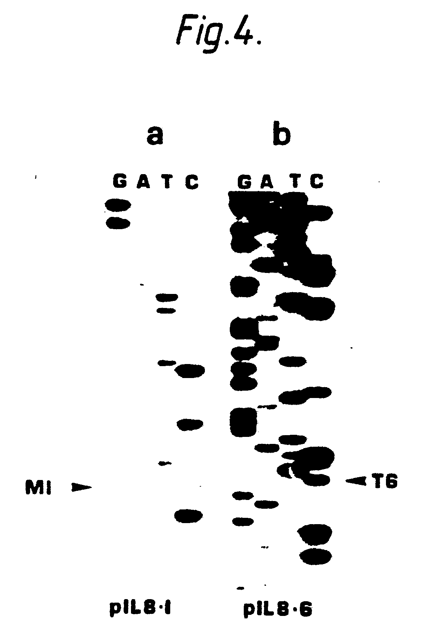

The mutagenesis experiments were performed using the same protocol described for the in frame positioning (Fig. 4A and B). For the double mutants we first obtained the T6 mutation and then added the MI mutation.

The resulting plasmids were named:

```
pIL5-6   = apoAI-T6 between two Bam HI sites
pIL5-I   = apoAI-MI  "      "    "    "    "
pIL5-6I  = apoAI-T6/MI between two Bam HI sites
pIL8-6   = apoAI-T6 in frame
pIL8-I   = apoAI-MI  "    "
pIL8-6I  = apoAI-T6/MI in frame
```

Construction of the variants apoAl-RP5 and IP1

The DNA fragments containing the apoAl gene and its variants T6 and MI were excised from plasmids pLS66, pIL5-6, pIL5-I and pIL5-6I by Bam HI digestion and were purified on a 1% agarose gel as described. The fragments were then ligated to pUC8 and pUC9 (Vieira and Messing, Gene, 19: 259-268, 1982), both linearized with Bam HI. After transformation of JM101 competent cells, recombinant plasmids were identified by the rapid disruption method and verified by cutting isolates with Bam HI. Two opposite orientations of the insert were distinguished by cutting with Hind III and Xho I.

Constructions with pUC8 had the apoAl gene already in frame, immediately preceeded by a methionine residue and the first 9 amino acids of the alpha-peptide from the bacterial beta-galactosidase gene; this resulted in a 10 amino acid extension at the N-terminal of the apoAl molecule of beta-galatosidase.

All the recombinant pUC9 derivatives, containing the apoAl genes in the correct orientation, required more work to put the apoAl gene in frame. They were first cut with the restriction enzymes Hind III and Sal I between the ATG translation start and the apoAl gene. The cohesive ends generated by cutting were filled in with DNA polymerase (Klenow fragment) and then religated with T4 DNA ligase. This resulted in the deletion of 10 nucleotides at the multiple cloning site of pUC9 , in front of the apoAl gene, thus restoring the correct reading frame.

The recombinant plasmids were named:

```
a)  pUC8 series
pIPI       = with apoAI-IPI
pIPI-6   = "      "      "    /T6
pIPI-I   = "      "      "    /MI
pIPI-6I  = "      "      "    /T6/MI
b)  pUC9 series
pRP5       = with apoAI-RP5
pRP5-6   = "      "      "    /T6
pRP5-I   = "      "      "    /MI
pRP5-6I  = "      "      "    /T6/MI
```

The rationale to obtain the extended variants was that expression levels were increased by the presence of the N-terminal end of bacterial beta-galactosidase, the sequence naturally occurring after the regulatory elements present in the plasmids.

Expression in E. coli strains

1) All recombinant pFCE4 derivatives were transformed in the bacterial host CAG629 bearing the plasmid pCI856 (already described in the "In Frame Positioning" section).

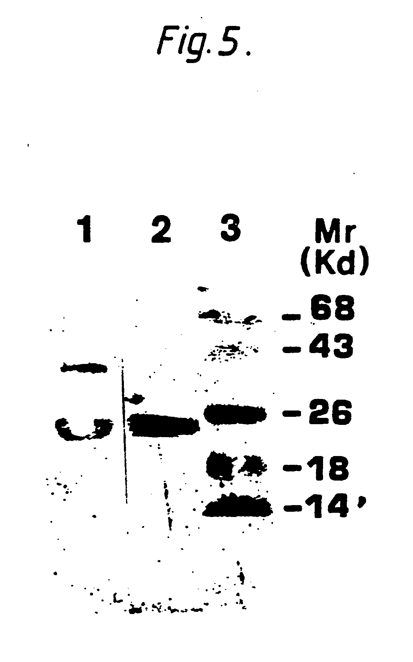

Overnight cultures grown at $32^\circ$ C were diluted 1:10 in LA and grown in shaker flasks till OD $_{650}$ 0.5-0.6, then the temperature was shifted at $42^\circ$ C and incubation was continued for 1-2 hours. After induction, cells were chilled on ice for 10 min. and pelleted by centrifugation. The pellets were then treated as described in the "ELISA" section to break the cells. The cell extracts were centrifuged at 16.000 rpm for 30 min. at 4° C and the supernatant applied to the affinity column described in "Materials and Methods". After extensive washing the retained proteins were eluted with 1M acetic acid. The eluted fractions were assayed by immunoblot analysis (Fig. 5) and by "ELISA"; preliminary results obtained with pML11-20 averaged around 0.2 mg of apoAI per liter of culture.

Similar results were obtained with all other constructions bearing mutations apoAI-T6, apoAI-MI and apoAI-T6/MI.

2) All recombinant pUC8/9 derivatives were transformed in the bacterial host MC1061.

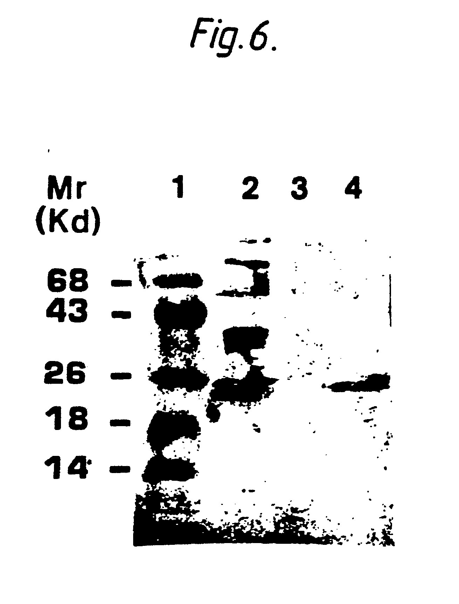

Overnight cultures grown at 37° C were diluted 1:100 in LA and grown in shaker flasks for 1 hour prior the addition of 1 mM IPTG, then incubation was continued for an other 4-6 hours. After this time the cells were chilled on ice for 10 min. and pelleted by centrifugation. From this step on, the extraction procedures were the same as those described for the pFCE4 derivatives. Crude extracts of cells containing pIPI and pRP5 were assayed by immunoblot analysis (Fig. 6) and by "ELISA"; preliminary results averaged around 10 mg of apoAI per liter of culture.

Similar results were obtained with all other constructions bearing mutations apoAI-T6, apoAI-MI and apoAI-T6/MI in combination with both apoAI-RP5 and apoAI-IP1.

Example 2

Human apoAI is synthesized as a precursor protein, preproapo AI of 267 amino acids. The 18 amino acids long prepeptide is cleaved during secretion, leaving a proprotein denoted proapoAI. ProapoAI consists therefore of a six aminoacids N-terminal extension, Arg-His-Phe-Trp-Gln-Gln, followed by the mature protein.

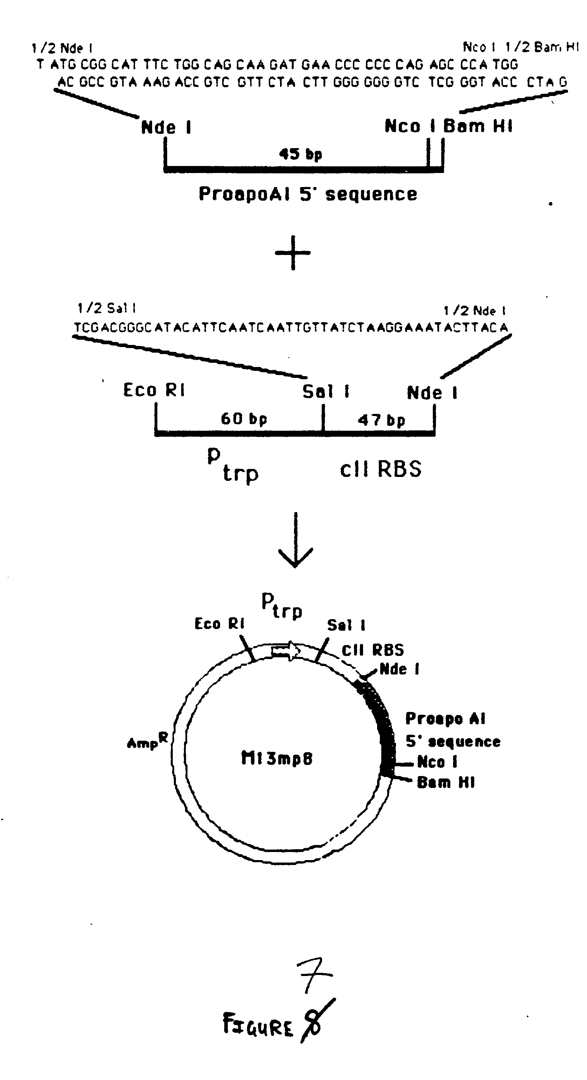

In order to express proapoAI, the 5' of the apoAI gene was synthetically reconstructed as shown in Figure 7. A Nde I-Bam HI oligonucleotide coding for the first 15 amino acids of proapo AI including the 6 amino acids propeptide was synthesized. The synthetic DNA included an Nco I site immediately before the Bam HI site. An Eco RI-Sal I fragment carrying the trp promoter from plasmid pDR720 (Pharmacia, Sweden; Russel and Bennet, Gene 20,231,1982) was ligated to a synthetic Sal I-Nde I fragment coding for the lambda cII Shine-Dalgarno sequence to obtain the Eco RI-Nde I 107 bp fragment illustrated in the figure. This DNA piece was ligated to the fragment coding for the proapoAI 5' sequence and subcloned into M13mp8 (Messing et al, Proc.Natl.Acad.Sci.USA 74,3642, 1977) to be sequenced.

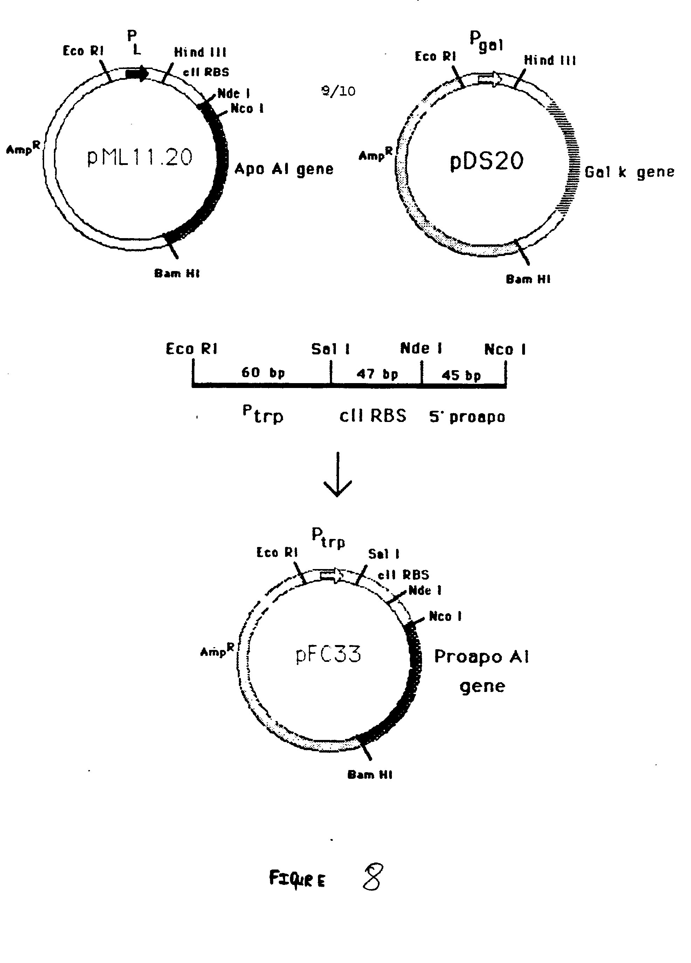

In order to express the recombinant proapoAI, an expression vector was constructed as illustrated in Figure 8. An Eco RI-Nco I fragment harbouring the expression signals followed by the 5' end of the proapoAI gene was cut from the recombinant M13mp8 (See Fig.7) and ligated to the two following fragments:

a) the large fragment of plasmid pDS20 (Duester et al, Cell 30, 855, 1982) cut with Eco RI-Bam HI

b) an Nco I-Bam HI fragment from pML11-20 containing the rest of the apoAI sequence.

The resulting plasmid pFC33 was able to express a proapoAI protein, or more specifically Met-proapoAI, under the trp promoter in an E. coli B strain (Delbruck, 1946, Bacterial viruses or bacteriophages Biol. Rev. 21:30-40). For induction, cells were grown overnight in a LB medium with ampicillin. The next day, cells were diluted into M9 medium without tryptophan and harvested after 6 hours at 37° C.

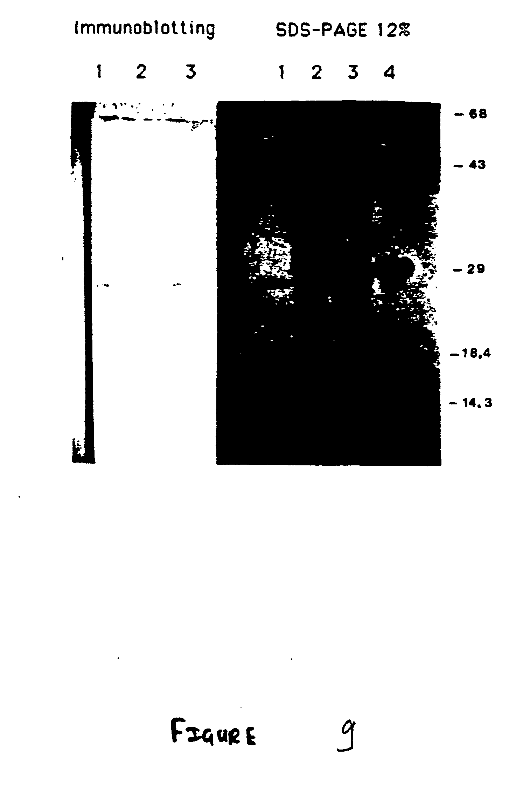

Figure 9 shows a gel electrophoresis and an immunoblot analysis of proapoAI. Aliquots of bacterial cultures were pelleted and resuspended with gel electrophoresis buffer in parallel with a standard human fraction. After boiling, samples were electrophoresed on 12.5% SDS-PAGE. Blotting on nitrocellulose filter was performed as described in Example 1.

TABLE 1

| ZYGOSIS | NAME | MODIFICATION | BIOCHEMICAL ALTERATIONS |
|---|---|---|---|
| HETEROZYGOTE | MILANO | $Arg_{173}$ --> Cys | FORMS DIMERS AND COMPLEXES WITH Apo AII |
| | GIESSEN | $Pro_{143}$ --> Arg | 60-70% OF NORMAL LCAT ACTIVATION ABILITY |
| | MARBURG (MUNSTER 2) | $Lys_{107}$ --> 0 | 40-60% OF NORMAL LCAT ACTIVATION ABILITY |
| | MUNSTER 3 : FAMILY A | $Asp_{103}$ --> Asn | NONE |
| | B | $Pro_{4}$ --> Arg | |
| | C | $Pro_{3}$ --> His | |

ApoAI genetic variants naturally occurring in the human population

## Claims

Claims for the following Contracting States: AT, BE, CH, DE, FR, GB, IT, LI, LU, NL, SE

1. An expression vector capable of expressing, in a transformed host, a protein which is capable of being

12

detected by ELISA with anti-human apoAI antiserum and which has the formula (1)

Met-X-Y   (1)

wherein x is -Thr-Met-Ile-Thr-Pro-Ser-Phe-Asp-Gly-Ser-Met-,-Thr-Met-Ile-Thr-Asn-Ser-Arg-Gly-Ser-Met- or the pro sequence of human apoAI; and Y represents the sequence of human apoAI or human apoAI in which the Arg at position 173 is substituted by Cys (apoAI-MI), in which the Ser at position 6 is substituted by Thr (apoAI-T6) or which incorporates both substitutions (apoAI-T6/MI)

2. An expression vector according to claim 1, wherein in the protein of formula (1) X is:
-Thr-Met-Ile-Thr-Pro-Ser-Phe-Asp-Gly-Ser-Met-
and which is plasmid pUC8 having the gene encoding apoAI, apoAI-T6, apoAI-MI or apoAI-T6/MI provided·at the Bam HI site immediately after the codon for the ninth amino acid of the alpha-peptide of the beta-galactosidase gene of the said plasmid.

3. An expression vector according to claim 1, wherein in the protein of formula (1) X is:
-Thr-Met-Ile-Thr-Asn-Ser-Arg-Gly-Ser-Met-
and which is plasmid pUC9 having the gene encoding apoAI, apoAI-T6, apoAI-MI or apoAI-T6/MI provided immediately after the codons for the sequence:
-Thr-Met-Ile-Thr-Asn-Ser-Arg-Gly-Ser-Met-
which are in turn provided immediately after the translational start codon provided by the plasmid.

4. An expression vector according to claim 1, which is a plasmid capable of expressing the said protein of formula (1) in which X is the pro sequence of human apoAI under the control of the trp promoter.

5. A host which has been transformed with an expression vector as defined in any one of the preceding claims and in which thereby a said protein of formula (1) is capable of being expressed.

6. A host according to claim 5, wherein the host is a strain of E. coli.

7. A process for producing a protein of formula (1) as defined in claim 1, which process comprises:
   (i) culturing a host as defined in claim 5 or 6; and
   (ii) recovering the said protein thus-obtained.

8. A protein of formula (1) as defined in claim 1.

9. A protein according to claim 8, wherein the amino acid sequence Met-Thr-Met-Ile-Thr-Pro-Ser-Phe-Asp-Gly-Ser-Met is fused to an apolipoprotein selected from apoAI, apoAI-T6, apoAI-MI and apoAI-T6/MI.

10. A protein according to claim 8 wherein the amino acid sequence Met-Thr-Met-Ile-Thr-Asn-Ser-Arg-Gly-Ser-Met is fused to an apolipoprotein selected from apoAI, apoAI-T6, apoAI-MI and apoAI-T6/MI.

11. A protein according to claim 8 which is Met-proapoAI.

12. A pharmaceutical composition comprising a protein as defined in claim 8 as active ingredient and a pharmaceutically acceptable carrier or diluent.

Claims for the following Contracting State: ES

1. A process for producing a protein which is capable of being detected by ELISA with anti-human apoAI antiserum and which has the formula (1)

Met-X-Y   (1)

wherein X is -Thr-Met-Ile-Thr-Pro-Ser-Phe-Asp-Gly-Ser-Met-,-Thr-Met-Ile-Thr-Asn-Ser-Arg-Gly-Ser-Met- or the pro sequence of human apoAI; and y represents the sequence of human apoAI or human apoAI in which the Arg at position 173 is substituted by Cys (apoAI-MI), in which the Ser at position 6 is substituted by Thr (apoAI-T6) or which incorporates both substitutions (apoAI-T6/MI); which process

13

EP 0 267 703 B1

comprises:

(i) culturing a host transformed with an expression vector capable of expressing the said protein in the said host; and

(ii) recovering the said protein thus obtained.

2. A process according to claim 1, wherein the protein of formula (1) in which X is:
-Thr-Met-Ile-Thr-Pro-Ser-Phe-Asp-Gly-Ser-Met-
is obtained by culturing a host transformed with plasmid pUC8 having the gene encoding apoAI, apoAI-T6, apoAI-MI or apoAI-T6/MI provided at the Bam HI site immediately after the codon for the ninth amino acid of the alpha-peptide of the beta-galactosidase gene of the said plasmid.

3. A process according to claim 1, wherein the protein of formula (1) in which X is:
-Thr-Met-Ile-Thr-Asn-Ser-Arg-Gly-Ser-Met-
is obtained by culturing a host transformed with plasmid pUC9 having the gene encoding apoAI, apoAI-T6, apoAI-MI or apoAI-T6/MI provided immediately after the codons for the sequence:
-Thr-Met-Ile-Thr-Asn-Ser-Arg-Gly-Ser-Met-
which are in turn provided immediately after the translational start codon provided by the plasmid.

4. A process according to claim 1, wherein the protein of formula (1) in which X is the pro sequence of human apoAI is expressed from a plasmid under the control of the trp promoter.

5. A process according to any one of the preceding claims, wherein the host is a strain of E. coli.

6. A process according to any one of the preceding claims, further comprising forming a pharmaceutical composition by formulating the protein of formula (1) thus obtained with a pharmaceutically acceptable carrier or diluent.

Claims for the following Contracting State: GR

1. An expression vector capable of expressing, in a transformed host, a protein which is capable of being detected by ELISA with anti-human apoAI antiserum and which has the formula (1)

Met-X-Y  (1)

wherein X is -Thr-Met-Ile-Thr-Pro-Ser-Phe-Asp-Gly-Ser-Met-,-Thr-Met-Ile-Thr-Asn-Ser-Arg-Gly-Ser-Met- or the pro sequence of human apoAI; and Y represents the sequence of human apoAI or human apoAI in which the Arg at position 173 is substituted by Cys (apoAI-MI), in which the Ser at position 6 is substituted by Thr (apoAI-T6) or which incorporates both substitutions (apoAI-T6/MI).

2. An expression vector according to claim 1, wherein in the protein of formula (1) X is:
-Thr-Met-Ile-Thr-Pro-Ser-Phe-Asp-Gly-Ser-Met-
and which is plasmid pUC8 having the gene encoding apoAI, apoAI-T6, apoAI-MI or apoAI-T6/MI provided at the Bam HI site immediately after the codon for the ninth amino acid of the alpha-peptide of the beta-galactosidase gene of the said plasmid.

3. An expression vector according to claim 1, wherein in the protein of formula (1) X is:
-Thr-Met-Ile-Thr-Asn-Ser-Arg-Gly-Ser-Met-
and which is plasmid pUC9 having the gene encoding apoAI, apoAI-T6, apoAI-MI or apoAI-T6/MI provided immediately after the codons for the sequence:
-Thr-Met-Ile-Thr-Asn-Ser-Arg-Gly-Ser-Met-
which are in turn provided immediately after the translational start codon provided by the plasmid.

4. An expression vector according to claim 1, which is a plasmid capable of expressing the said protein of formula (1) in which X is the pro sequence of human apoAI under the control of the trp promoter.

5. A host which has been transformed with an expression vector as defined in any one of the preceding claims and in which thereby a protein of formula (1) is capable of being expressed.

14

EP 0 267 703 B1

6. A host according to claim 5, wherein the host is a strain of E. coli.

7. A process for producing a protein of formula (1) as defined in claim 1, which process comprises:
   (i) culturing a host as defined in claim 5 or 6; and
   (ii) recovering the said protein thus-obtained.

8. A process according to claim 7, further comprising forming a pharmaceutical composition by formulating the protein of formula (1) thus-obtained with a pharmaceutically acceptable carrier or diluent.

**Revendications**

Revendications pour les Etats contractants suivants: AT, BE, CH, DE, FR, GB, IT, LI, LU, NL, SE

1. Vecteur d'expression capable d'exprimer, dans un hôte transformé, une protéine qui est susceptible d'être détectée par essai ELISA avec un antisérum anti-apoAl humaine et qui a la formule (1):

   Met - X - Y   (1)

   dans laquelle :
   - X représente -Thr-Met-Ile-Thr-Pro-Ser-Phe-Asp-Gly-Ser-Met-, -Thr-Met-Ile-Thr-Asn-Ser-Arg-Gly-Ser-Met- ou la proséquence d'apoAl humaine ; et
   - Y représente la séquence d'apoAl humaine ou d'apoAl humaine dans laquelle l'Arg en position 173 est substituée par Cys (apoAl-MI), dans laquelle la Ser en position 6 est substituée par Thr (apoAl-T6) ou qui incorpore les deux substitutions (apoAl-T6/MI).

2. Vecteur d'expression selon la revendication 1, dans lequel, dans la protéine de formule (1), X représente :
   -Thr-Met-Ile-Thr-Pro-Ser-Phe-Asp-Gly-Ser-Met-
   et qui est le plasmide pUC8 ayant le gène codant pour apoAl, apoAl-T6, apoAl-MI ou apoAl-T6/MI, disposé sur le site Bam HI immédiatement après le codon dudit plasmide pour le neuvième acide aminé de l'alpha-peptide du gène de la bêtagalactosidase.

3. Vecteur d'expression selon la revendication 1, dans lequel, dans la protéine de formule (1), X représente :
   -Thr-Met-Ile-Thr-Asn-Ser-Arg-Gly-Ser-Met-
   et qui est le plasmide pUC9 ayant le gène codant pour apoAl, apoAl-T6, apoAl-MI ou apoAl-T6/MI, disposé immédiatement après les codons pour la séquence :
   -Thr-Met-Ile-Thr-Asn-Ser-Arg-Gly-Ser-Met-
   qui sont à leur tour disposés immédiatement après le codon d'initiation de la traduction, fourni par le plasmide.

4. Vecteur d'expression selon la revendication 1, qui est un plasmide capable d'exprimer ladite protéine de formule (1), dans laquelle X représente la proséquence d'apoAl humaine, sous le contrôle du promoteur trp.

5. Hôte qui a été transformé par un vecteur d'expression tel que défini à l'une des revendications précédentes et dans lequel, de cette façon, une protéine de formule (1) est capable d'être exprimée.

6. Hôte selon la revendication 5, dans lequel l'hôte est une souche d'E. coli.

7. Procédé de production d'une protéine de formule (1) telle que définie à la revendication 1, lequel procédé comprend :
   (i) la culture d'un hôte tel que défini à la revendication 5.ou 6 ; et
   (ii) la récupération de ladite protéine ainsi obtenue.

8. Protéine de formule (1) telle que définie à la revendication 1.

9. Protéine selon la revendication 8, dans laquelle la séquence d'acides aminés
   Met-Thr-Met-Ile-Thr-Pro-Ser-Phe-Asp-Gly-Ser-Met

15

EP 0 267 703 B1

est fusionnée à une apolipoprotéine choisie parmi apoAI, apoAI-T6, apoAI-MI et apoAI-T6/MI.

**10.** Protéine selon la revendication 8, dans laquelle la séquence d'acides aminés
Met-Thr-Met-Ile-Thr-Asn-Ser-Arg-Gly-Ser-Met
est fusionnée à une apolipoprotéine choisie parmi apoAI, apoAI-T6, apoAI-MI et apoAI-T6/MI.

**11.** Protéine selon la revendication 8, qui est Met-proapoAI.

**12.** Composition pharmaceutique comprenant une protéine telle que définie à la revendication 8 comme ingrédient actif et un support ou diluant pharmaceutiquement acceptable.

Revendications pour l'Etat contractant suivant: ES

**1.** Procédé de production d'une protéine qui est susceptible d'être détectée par essai ELISA avec un antisérum anti-apoAI humaine et qui a la formule (1):

Met-X-Y   (1)

dans laquelle :
- X représente -Thr-Met-Ile-Thr-Pro-Ser-phe-Asp-Gly-Ser-Met-, -Thr-Met-Ile-Thr-Asn-Ser-Arg-Gly-Ser-Met- ou la proséquence d'apoAI humaine ; et
- Y représente la séquence d'apoAI humaine ou d'apoAI humaine dans laquelle l'Arg en position 173 est substituée par Cys (apoAI-MI), dans laquelle la Ser en position 6 est substituée par Thr (apoAI-T6) ou qui incorpore les deux substitutions (apoAI-T6/MI),
lequel procédé comprend :
(i) la culture d'un hôte transformé par un vecteur d'expression capable d'exprimer ladite protéine dans ledit hôte ; et
(ii) la récupération de ladite protéine ainsi obtenue.

**2.** Procédé selon la revendication 1, dans lequel, la protéine de formule (1) dans laquelle X représente :
-Thr-Met-Ile-Thr-Pro-Ser-Phe-Asp-Gly-Ser-Met-
est obtenue par culture d'un hôte transformé avec le plasmide pUC8 ayant le gène codant pour apoAI, apoAI-T6, apoAI-MI ou apoAI-T6/MI, disposé sur le site Bam HI immédiatement après le codon dudit plasmide pour le neuvième acide aminé de l'alpha-peptide du gène de la bêtagalactosidase.

**3.** Procédé selon la revendication 1, dans lequel la protéine de formule (1) dans laquelle X représente :
-Thr-Met-Ile-Thr-Asn-Ser-Arg-Gly-Ser-Met-
est obtenuepar culture d'un hôte transformé avec le plasmide pUC9 ayant le gène codant pour apoAI, apoAI-T6, apoAI-MI ou apoAI-T6/MI, disposé immédiatement après les codons pour la séquence :
-Thr-Met-Ile-Thr-Asn-Ser-Arg-Gly-Ser-Met-
qui sont à leur tour disposés immédiatement après le codon d'initiation de la traduction, fourni par le plasmide.

**4.** Procédé selon la revendication 1, dans lequel la protéine de formule (1), dans laquelle X représente la proséquence d'apoAI humaine, est exprimée à partir d'un plasmide sous le contrôle du promoteur trp.

**5.** Procédé selon l'une des revendications précédentes, dans lequel l'hôte est une souche d'E. coli.

**6.** Procédé selon l'une des revendications précédentes, comprenant en outre la formation d'une composition pharmaceutique par formulation de la protéine de formule (1) ainsi obtenue avec un support ou diluant pharmaceutiquement acceptable.

Revendications pour l'Etat contractant suivant: GR

**1.** Vecteur d'expression capable d'exprimer, dans un hôte transformé, une protéine qui est susceptible d'être détectée par essai ELISA avec un antisérum anti-apoAI humaine et qui a la formule (1):

Met-X-Y   (1)

16

dans laquelle :
- X représente -Thr-Met-Ile-Thr-Pro-Ser-Phe-Asp-Gly-Ser-Met-, -Thr-Met-Ile-Thr-Asn-Ser-Arg-Gly-Ser-Met- ou la proséquence d'apoAI humaine ; et
- Y représente la séquence d'apoAI humaine ou d'apoAI humaine dans laquelle l'Arg en position 173 est substituée par Cys (apoAI-MI), dans laquelle la Ser en position 6 est substituée par Thr (apoAI-T6) ou qui incorpore les deux substitutions (apoAI-T6/MI).

2. Vecteur d'expression selon la revendication 1, dans lequel, dans la protéine de formule (1), X représente :
-Thr-Met-Ile-Thr-Pro-Ser-Phe-Asp-Gly-Ser-Met-
et qui est le plasmide pUC8 ayant le gène codant pour apoAI, apoAI-T6, apoAI-MI ou apoAI-T6/MI, disposé sur le site Bam HI immédiatement après le codon dudit plasmide pour le neuvième acide aminé de l'alpha-peptide du gène de la bêtagalactosidase.

3. Vecteur d'expression selon la revendication 1, dans lequel, dans la protéine de formule (1), X représente :
-Thr-Met-Ile-Thr-Asn-Ser-Arg-Gly-Ser-Met-
et qui est le plasmide pUC9 ayant le gène codant pour apoAI, apoAI-T6, apoAI-MI ou apoAI-T6/MI, disposé immédiatement après les codons pour la séquence :
-Thr-Met-Ile-Thr-Asn-Ser-Arg-Gly-Ser-Met-
qui sont à leur tour disposés immédiatement après le codon d'initiation de la traduction, fourni par le plasmide.

4. Vecteur d'expression selon la revendication 1, qui est un plasmide capable d'exprimer ladite protéine de formule (1), dans laquelle X représente la proséquence d'apoAI humaine, sous le contrôle du promoteur trp.

5. Hôte qui a été transformé par un vecteur d'expression tel que défini à l'une des revendications précédentes et dans lequel, de cette façon, une protéine de formule (1) est capable d'être exprimée.

6. Hôte selon la revendication 5, dans lequel l'hôte est une souche d'E. coli.

7. Procédé de production d'une protéine de formule (1) telle que définie à la revendication 1, lequel procédé comprend :
(i) la culture d'un hôte tel que défini à la revendication 5 ou 6 ; et
(ii) la récupération de ladite protéine ainsi obtenue.

8. Procédé selon la revendication 7, comprenant en outre la formation d'une composition pharmaceutique par formulation de la protéine de formule (1) ainsi obtenue avec un support ou diluant pharmaceutiquement acceptable.

**Ansprüche**
Patentansprüche für folgende Vertragsstaaten: AT, BE, CH, DE, FR, GB, IT, LI, LU, NL, SE

1. Expressionsvektor, der zur Expression eines Proteins in einer transformierten Wirtszelle in der Lage ist, wobei das Protein durch ELISA mit anti-Mensch-apoAI-Antiserum nachgewiesen werden kann, und das Protein die Formel

Met-X-Y  (1)

hat,
worin X -Thr-Met-Ile-Thr-Pro-Ser-Phe-Asp-Gly-Ser-Met-, -Thr-Met-Ile-Thr-Asn-Ser-Arg-Gly-Ser-Met- oder die Prosequenz des menschlichen apoAI ist; und Y die Sequenz des menschlichen apoAI oder menschlichen apoAI ist, bei dem das Arg an Position 173 durch Cys (apoAI-MI) substituiert ist, bei dem das Ser an Position 6 durch Thr (apoAI-T6) substituiert ist, oder welches beide Substitutionen (apoAi-T6/MI) enthält.

2. Expressionsvektor nach Anspruch 1, worin X im Protein der Formel (1)
-Thr-Met-Ile-Thr-Pro-Ser-Phe-Asp-Gly-Ser-Met-
ist, und der Expressionsvektor das Plasmid pUC8 ist, bei der das apoAl, apoAl-T6, apoAl-MI oder apoAl-T6/MI kodierende Gen in die Bam HI Schnittstelle unmittelbar nach dem Kodon für die neunte Aminosäure des alpha-Peptids des beta-Galactosidasegens auf dem Plasmid eingeschleust wurde.

3. Expressionsvektor nach Anspruch 1, worin X im Protein der Formel (1)
-Thr-Met-Ile-Thr-Asn-Ser-Arg-Gly-Ser-Met-
ist, und der Vektor das Plasmid pUC9 ist, bei dem das apoAl, apoAl-T6, apoAl-MI oder apoAl-T6/MI kodierende Gen unmittelbar nach den Kodons für die Sequenz -Thr-Met-Ile-Thr-Asn-Ser-Arg-Gly-Ser-Met-eingeschleust wurde, die ihrerseits unmittelbar nach dem translationalen Startkodon auf dem Plasmid eingeschleust wurden.

4. Expressionsvektor nach Anspruch 1, der ein Plasmid ist, das zur Expression des Proteins der Formel (1), bei dem X die Prosequenz des menschlichen apoAl ist, unter der Kontrolle der trp Promoters in der Lage ist.

5. Wirtszelle, die mit einem Expressionsvektor, wie nach einem der vorhergehenden Ansprüche definiert, transformiert wurde und in der hierdurch das Protein der Formel (1) exprimiert werden kann.

6. wirtszelle nach Anspruch 5, worin die Wirtszelle ein E. coli Stamm ist.

7. Verfahren zur Gewinnung eines Proteins der Formel (1), wie in Anspruch 1 definiert, umfassend:
(i) Kultur einer Wirtszelle wie in Anspruch 5 oder 6 definiert und
(ii) Gewinnung des so erhaltenen Proteins.

8. Protein der Formel (1) wie in Anspruch 1 definiert.

9. Protein nach Anspruch 8, worin die Aminosäuresequenz Met-Thr-Met-Ile-Thr-Pro-Ser-Phe-Asp-Gly-Ser-Met an ein Apolipoprotein, ausgewählt aus apoAl, apoAl-T6, apoAl-MI und apoAl-T6/MI, fusioniert ist.

10. Protein nach Anspruch 8, worin die Aminosäuresequenz Met-Thr-Met-Ile-Thr-Asn-Ser-Arg-Gly-Ser-Met an ein Apoliporotein, ausgewählt aus apoAl, apoAl-T6, apoAl-MI und apoAl-T6/MI, fusioniert ist.

11. Protein nach Anspruch 8, welches Met-proapoAl ist.

12. Pharmazeutische Zusammensetzung enthaltend ein Protein, wie in Anspruch 8 definiert, als wirksamen Bestandteil und einen pharmazeutisch annehmbaren Träger oder Verdünner.

Patentansprüche für folgenden Vertragsstaaten: ES

1. Verfahren zur Herstellung eines Proteins, das durch ELISA mit anti-Mensch-apoAl-Antiserum nachgewiesen werden kann und die Formel (1)

Met-X-Y  (1)

hat,
worin  X  -Thr-Met-Ile-Thr-Pro-Ser-Phe-Asp-Gly-Ser-Met-,  -Thr-Met-Ile-Thr-Asn-Ser-Arg-Gly-Ser-Met- oder die Prosequenz des menschlichen apoAl ist; und Y die Sequenz des menschlichen apoAl oder menschlichen apoAl ist, bei dem das Arg an Position 173 durch Cys (apoAl-MI) substituiert ist, bei dem das Ser an Position 6 durch Thr (apoAl-T6) substituiert ist, oder welches beide Substitutionen (apoAi-T6/MI) enthält, umfassend
(i) Kultur einer Wirtszelle, die mit einem Expressionsvektor, der zur Expression des Proteins in der Wirtszelle in der Lage ist, transformiert wurde und
(ii) Gewinnung des so erhaltenen Proteins.

2. Verfahren nach Anspruch 1, worin das Protein der Formel (1), bei dem X -Thr-Met-Ile-Thr-Pro-Ser-Phe-Asp-GlY-Ser-Met-ist, erhalten wird durch Kultur einer mit dem Plasmid pUC8 transformierten Wirtszelle,

bei der das apoAl, apoAl-T6, apoAl-Ml oder apoAl-T6/Ml kodierende Gen in die Bam HI Schnittstelle unmittelbar nach dem Kodon für die neunte Aminosäure des alpha-Peptids des beta-Galactosidasegens auf dem Plasmid eingeschleust wurde.

3. Verfahren nach Anspruch 1, worin das Protein der Formel (1), bei dem X -Thr-Met-Ile-Thr-Asn-Ser-Arg-Gly-Ser-Met-ist, erhalten wird durch Kultur einer mit dem Plasmid pUC9 transformierten Wirtszelle, bei dem das apoAl, apoAl-T6, apoAl-Ml oder apoAl-T6/Ml kodierende Gen unmittelbar nach den Kodons für die Sequenz -Thr-Met-Ile-Thr-Asn-Ser-Arg-Gly-Ser-Met-eingeschleust wurde, die ihrerseits unmittelbar nach dem translationalen Startkodon auf dem Plasmid eingeschleust wurden.

4. Verfahren nach Anspruch 1, worin das Protein der Formel (1), bei dem X die Prosequenz des menschlichen apoAl ist, von einem Plasmid unter Kontrolle des trp Promoters exprimiert wird.

5. Verfahren nach einem der vorhergehenden Ansprüche, worin die Wirtszelle ein E. coli Stamm ist.

6. Verfahren nach einem der vorhergehenden Ansprüche umfassend als zusätzlichen Schritt die Herstellung einer pharmazeutischen Zusammensetzung durch Formulierung des so erhaltenen Proteins der Formel (1) mit einem pharmazeutisch annehmbaren Träger oder Verdünner.

Patentansprüche für folgenden Vertragsstaaten: GR

1. Expressionsvektor, der zur Expression eines Proteins in einer transformierten Wirtszelle in der Lage ist, wobei das Protein durch ELISA mit anti-Mensch-apoAl-Antiserum nachgewiesen werden kann, und das Protein die Formel

Met-X-Y   (1)

hat,
worin   X  -Thr-Met-Ile-Thr-Pro-Ser-Phe-Asp-Gly-Ser-Met-,   -Thr-Met-Ile-Thr-Asn-Ser-Arg-Gly-Ser-Met- oder die Prosequenz des menschlichen apoAl ist; und Y die Sequenz des menschlichen apoAl oder menschlichen apoAl ist, bei dem das Arg an Position 173 durch Cys (apoAl-Ml) substituiert ist, bei dem das Ser an Position 6 durch Thr (apoAl-T6) substituiert ist, oder welches beide Substitutionen (apoAi-T6/Ml) enthält.

2. Expressionsvektor nach Anspruch 1, worin im Protein der Formel (1)
-Thr-Met-Ile-Thr-Pro-Ser-Phe-Asp-Gly-Ser-Met-
ist, und der Expressionsvektor das Plasmid pUC8 ist, bei der das apoAl, apoAl-T6, apoAl-Ml oder apoAl-T6/Ml kodierende Gen in die Bam HI Schnittstelle unmittelbar nach dem Kodon für die neunte Aminosäure des alpha-Peptids des beta-Galactosidasegens auf dem Plasmid eingeschleust wurde.

3. Expressionsvektor nach Anspruch 1, worin X im Protein der Formel (1)
-Thr-Met-Ile-Thr-Asn-Ser-Arg-Gly-Ser-Met-
ist, und der Vektor das Plasmid pUC9 ist, bei dem das apoAl, apoAl-T6, apoAl-Ml oder apoAl-T6/Ml kodierende Gen unmittelbar nach den Kodons für die Sequenz -Thr-Met-Ile-Thr-Asn-Ser-Arg-Gly-Ser-Met-eingeschleust wurde, die ihrerseits unmittelbar nach dem translationalen Startkodon auf dem Plasmid eingeschleust wurden.

4. Expressionsvektor nach Anspruch 1, der ein Plasmid ist, das zur Expression des Proteins der Formel (1), bei dem X die Prosequenz des menschlichen apoAl ist, unter der Kontrolle der trp Promoters in der Lage ist.

5. Wirtszelle, die mit einem Expressionsvektor, wie nach einem der vorhergehenden Ansprüche definiert, transformiert wurde und in der hierdurch das Protein der Formel (1) exprimiert werden kann.

6. Wirtszelle nach Anspruch 5, worin die Wirtszelle ein E. coli Stamm ist.

7. Verfahren zur Gewinnung eines Proteins der Formel (1), wie in Anspruch 1 definiert, umfassend:
(i) Kultur einer Wirtszelle wie in Anspruch 5 oder 6 definiert und

(ii) Gewinnung des so erhaltenen Proteins.

8. Verfahren nach Anspruch 7 umfassend als zusätzlichen Schritt die Herstellung einer pharmazeutischen Zusammensetzung durch Formulierung des so erhaltenen Proteins der Formel (1) mit einem pharmazeutisch annehmbaren Träger oder Verdünner.

# Fig.1.

## APOA1

```
                              27                                    54
GAT GAA CCC CCC CAG AGC CCC TGG GAT CGA GTG AAG GAC CTG GCC ACT GTG TAC
Asp Glu Pro Pro Gln Ser Pro Trp Asp Arg Val Lys Asp Leu Ala Thr Val Tyr


                              81                                    108
GTG GAT GTG CTC AAA GAC AGC GGC AGA GAC TAT GTG TCC CAG TTT GAA GGC TCC
Val Asp Val Leu Lys Asp Ser Gly Arg Asp Tyr Val Ser Gln Phe Glu Gly Ser


                              135                                   162
GCC TTG GGA AAA CAG CTA AAC CTA AAG CTC CTT GAC AAC TGG GAC AGC GTG ACC
Ala Leu Gly Lys Gln Leu Asn Leu Lys Leu Leu Asp Asn Trp Asp Ser Val Thr


                              189                                   216
TCC ACC TTC AGC AAG CTG CGC GAA CAG CTC GGC CCT GTG ACC CAG GAG TTC TGG
Ser Thr Phe Ser Lys Leu Arg Glu Gln Leu Gly Pro Val Thr Gln Glu Ph Trp


                              243                                   270
GAT AAC CTG GAA AAG GAG ACA GAG GGC CTG AGG CAG GAG ATG AGC AAG GAT CTG
Asp Asn Leu Glu Lys Glu Thr Glu Gly Leu Arg Gln Glu MET Ser Lys Asp Leu


                              297                                   324
GAG GAG GTG AAG GCC AAG GTG CAG CCC TAC CTG GAC GAC TTC CAG AAG AAG TGG
Glu Glu Val Lys Ala Lys Val Gln Pro Tyr Leu Asp Asp Phe Gln Lys Lys Trp


                              351                                   378
CAG GAG GAG ATG GAG CTC TAC CGC CAG AAG GTG GAG CCG CTG CGC GCA GAG CTC
Gln Glu Glu MET Glu Leu Tyr Arg Gln Lys Val Glu Pro Leu Arg Ala Glu Leu


                              405                                   432
CAA GAG GGC GCG CGC CAG AAG CTG CAC GAG CTG CAA GAG AAG CTG AGC CCA CTG
Gln Glu Gly Ala Arg Gln Lys Leu His Glu Leu Gln Glu Lys Leu Ser Pro Leu


                              459                                   486
GGC GAG GAG ATG CGC GAC CGC GCG CGC GCC CAT GTG GAC GCG CTG CGC ACG CAT
Gly Glu Glu MET Arg Asp Arg Ala Arg Ala His Val Asp Ala Leu Arg Thr His


                              513                                   540
CTG GCC CCC TAC AGC GAC GAG CTG CGC CAG CGC TTG GCC GCG CGC CTT GAG GCT
Leu Ala Pro Tyr Ser Asp Glu Leu Arg Gln Arg Leu Ala Ala Arg Leu Glu Ala


                              567                                   594
CTC AAG GAG AAC GGC GGC GCC AGA CTG GCC GAG TAC CAC GCC AAG GCC ACC GAG
Leu Lys Glu Asn Gly Gly Ala Arg Leu Ala Glu Tyr His Ala Lys Ala Thr Glu


                              621                                   648
CAT CTG AGC ACG CTC AGC GAG AAG GCC AAG CCC GCG CTC GAG GAC CTC CGC CAA
His Leu Ser Thr Leu Ser Glu Lys Ala Lys Pro Ala Leu Glu Asp Leu Arg Gln


                              675                                   702
GGC CTG CTG CCC GTG CTG GAG AGC TTC AAG GTC AGC TTC CTG AGC GCT CTC GAG
Gly Leu Leu Pro Val Leu Glu Ser Phe Lys Val Ser Phe Leu Ser Ala Leu Glu


                              729
GAG TAC ACT AAG AAG CTC AAC ACC CAG TGA
Glu Tyr Thr Lys Lys Leu Asn Thr Gln
```

## Fig.2.

EcoRI                                                    Bam HI
pAT 153-Pvu II8                Apo AI coding sequence              pAT 153-Pvu II8

↓ EcoRI, Bam HI

EcoRI Sau3AI      Sau3AI                        Bam HI

O

Sau 3AI partial digestion,
ligation with 32 bp adapter,
recovery of 790 bp band

1                    2

Met Asp Glu Pro Pro Gln Ser Pro Trp
GATCCATGGACGAGCCACCGCAGAGTCCATGG
    GTACCTGCTCGGTGGCGTCTCAGGTACCCTAG

3                    4

Bam HI Sau3AI    Sau3AI                   Bam HI

# Fig.3.

pLS66

pML11-20

Fig.4.

# *Fig.5.*

Fig.6.

1/2 Nde I                                                         Nco I  1/2 Bam HI

T ATG CGG CAT TTC TGG CAG CAA GAT GAA CCC CCC CAG AGC CCA TGG

   AC GCC GTA AAG ACC GTC GTT CTA CTT GGG GGG GTC TCG GGT ACC CTA G

**Nde I**                                      **Nco I Bam HI**

**45 bp**

**ProapoAl 5' sequence**

**+**

1/2 Sal I                                                    1/2 Nde I

TCGACGGGCATACATTCAATCAATTGTTATCTAAGGAAATACTTACA

**Eco RI**                        **Sal I**        **Nde I**

**60 bp**           **47 bp**

$P_{trp}$           **cll RBS**

$P_{trp}$

**Eco RI**        **Sal I**

                 **Cll RBS**

                    **Nde I**

**Proapo Al**

**Amp$^R$**       **M13mp8**     **5' sequence**

                            **Nco I**

                            **Bam HI**

FIGURE 8

FIGURE 8

Immunoblotting    SDS-PAGE 12%

1   2   3        1   2   3   4

— 68

— 43

— 29

—18.4

— 14.3

FIGURE    9